(19) Europäisches Patentamt
European Patent Office
Office européen des brevets

(11) Publication number : 0 262 191 B1

# (12) EUROPEAN PATENT SPECIFICATION

(45) Date of publication of patent specification : 04.12.91 Bulletin 91/49

(51) Int. Cl.[5] : C07K 5/08, C12Q 1/56

(21) Application number : 87902173.1

(22) Date of filing : 19.03.87

(86) International application number : PCT/SE87/00141

(87) International publication number : WO 87/05608 24.09.87 Gazette 87/21

## (54) NOVEL PEPTIDE DERIVATIVES.

(30) Priority : 21.03.86 SE 8601327

(43) Date of publication of application : 06.04.88 Bulletin 88/14

(45) Publication of the grant of the patent : 04.12.91 Bulletin 91/49

(84) Designated Contracting States : AT BE CH DE FR GB IT LI LU NL SE

(56) References cited :
EP-A- 034 122
EP-A- 0 110 306
EP-A- 0 170 797
DE-A- 3 428 543

(73) Proprietor : Kabi Diagnostica AB
Taljegardsgatan 3
S-431 53 Molndal (SE)

(72) Inventor : ARIELLY, Salo
Violgatan 3A
S-434 00 Kungsbacka (SE)
Inventor : GUSTAVSSON, Stig, Ake, Ingemar
Vaktelgatan 15
S-431 33 Mölndal (SE)

(74) Representative : Widén, Björn et al
Kabi Pharmacia AB Patent Department
S-751 82 Uppsala (SE)

EP 0 262 191 B1

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

### Technical field

The present invention relates to novel tripeptide derivatives useful for determination of serine proteases. The novel derivatives are especially suitable for determination of Factor $X_a$ (E.C.3.4.21.6) or for analyses of reactions, in which $FX_a$ is formed, inhibited or consumed which makes it possible to quantify other enzymes and/or inhibitors present in the reactions.

### The background of the invention

Factor $X_a$ is a key substance in the reaction cascade which leads to the blood coagulation. Several methods based on the determination of $FX_a$ with chromogenic substrate have shown to be of considerable value within the clinical diagnostic in explanation of disturbances in the blood coagulation system (Hemker H.C. - Handbook of synthetic substrates, 1983, Martinus Nijhoff Publishers, Boston).

### Prior art

Chromogenic tetrapeptide derivatives are described by Aurell, L., et al. (Haemostasis vol. 7, 1978, 92-94) and have shown to be of considerable value in this case. These substrates are based on the amino acid sequence (-Ile-Glu-Gly-Arg-), which precedes the splitting places in the natural substrate prothrombin. The tetrapeptide substrates are characterized by high selectivity i.e. other enzymes, especially thrombin, do not disturb the determination of Factor $X_a$. Disadvantages are however the limited solubility and the several steps in the synthesis. EP 34 122 shows that tripeptide derivatives may be useful as $FX_a$ substrates. However, these tripeptides are sensitive to thrombin too. The high selectivity of the tetrapeptides is not obtained by these tripeptides.

EP 170 797 discloses compounds, which differ from our claimed compounds in that D-Arg is substituted with D-Lys. The inventive idea of EP 170 797 lies in a new marker 3-carboxy-4-hydroxyanilide as a chromogenic group.

The best compound among the claimed substrates EP 170 797 (PS 2000) shows lower activity compared with S-2222. Substrates according to this invention show 6-13 times higher activity compared with S-2222 for FXa.

### Description of the invention

The tripeptide derivatives in the present invention show high sensitivity and solubility and simultaneously selectivity properties comparable with the tetrapeptides in the prior art. The novel substrates are characterized by the following formula:

$$R_1 - X - D\text{-}Arg - A - Arg - NH - R_2$$

wherein $R_1$ is hydrogen, - or β-naphtyl residue, lower alkyl residue which may be substituted with a carboxyl group, unsubstituted or substituted phenyl- or phenylalkyl residue, wherein the alkyl group having 1 to 4 carbon atoms and preferably wherein the substitution is in para position in the phenyl ring and the substituents are lower alkyl, lower alkoxy, halogen or nitro group;

lower alkyl is a straight or branched alkyl group having 1 to 4 carbon atoms, preferably methyl, ethyl or tert. butyl;

lower alkoxy is an alkoxy group having 1 to 4 carbon atoms, preferably methoxy;

halogen is chlorine, bromine, fluorine, or iodine, preferably chlorine;

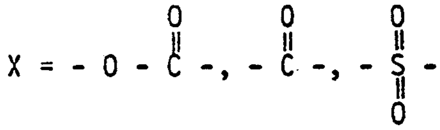

or a single bond with the proviso that

when $R_1$ is a hydrogen then and only then X is a single bond

A = Gly or Sar

$R_2$ = an aromatic or heterocyclic residue which gives the compound

$R_2 - NH_2$ by enzymatic hydrolysis

or di- and tri-salts of inorganic or organic acids thereof.

These compounds $R_2$ - $NH_2$ are prior known compounds with chromogenic, fluorogenic or electrochemical properties which permit quantifying of serine proteases by determination of splitted marker directly or after derivatization (Hemker H.C., loc. cit. and references cited therein).

Examples of compounds $R_2$ - $NH_2$ are:

p-nitroaniline, 3-carboxy-4-hydroxyaniline, 3-sulfo-4-nitroaniline, 3-alkoxy-4-nitroaniline, 3-carboxy-4-nitroaniline, 4-methoxy-β-naphtylamine, 4-(N-ethyl-N--hydroxyethyl)aminoaniline, 5-amino-isophtalic acid-dimethyl ester, 5-amino-8-nitroquinoline, 7-amino-4-trifluoromethyl coumarine, 7-amino-4-methyl coumarine, 4-aminodiphenylamine.

The novel derivatives containing two amino acids with strong basic side-chain can form disalts or when $R_1$ is hydrogen trisalts with inorganic or organic acids. Preferred salts are disalts of inorganic or organic acids since they possess a high water solubility. Especially preferred salts are hydrochlorides.

The splitting of the substrates by the appropriate enzymes for determination takes place on the carboxylic side of arginine in L-form, therefore the N-terminal arginine must be as D-isomer. Surprisingly, at biological pH the negatively charged or neutral lipophilic part of the tetrapeptide substrate could be changed to a positively charged strong basic amino acid with retained high selectivity and increased sensitivity.

The use of the peptide derivatives according to the present invention in methods for quantitative and/or qualitative determination of serine proteases or quantitative and/or qualitative determination of components, which can interact with serine proteases or zymogen forms thereof, such as Factor X which is activated to Factor $X_a$, are important applications.

The novel Factor $X_a$ substrates constitute by their sensitivity, high solubility and good selectivity a valuable addition at the establishment of diagnostic analyses based on determination of Factor $X_a$. A good selectivity is an important critirion in determination of $FX_a$ in plasma, since there is otherwise a risk of influence from thrombin which may be present initially or formed during the assay. Examples of important applications are determination of $FX_a$ in human plasma, after activation with a protease from Russel's Viper Venom (RVV-X), of antifactor $X_a$, of heparin, and of FVIII:C at haemophilia (Hemker H.C., loc. cit., S. Rosén, Scand. J. Haematol. Suppl. 40, vol. 33, 1984, 139-145). Especially important applications are analyses in connection with anticoagulantia treatment with low molecular weight heparin fractions, where traditional clot-based methods cannot be used. (Walenga J.M. et al., Seminars in Thrombosis and Hemostatis, vol. 11, no. 2, 1985, 100-107). Specifically included are methods relying upon determination of $FX_a$-activity and used for the direct determination of FX and $FX_a$ or for the indirect determination of FVII, $FVII_a$, FVIII:C, FVIII:$C_a$, FIX, $FIX_a$, antithrombin III, platelet factor 4, heparin, low molecular weight heparins and heparinoids. The properties of the novel substrates make them especially suitable for use in automatic analysers.

Description of synthesis

At the synthesis of the novel $FX_a$ substrates conventional protecting groups and coupling methods within the peptide chemistry are used (M. Bodansky: "Principles of Peptide Synthesis", Springer Verlag 1984), e.g. addition step-by-step of the amino acids at the C-terminal amino acid provided with a marker, or synthesis of the N-terminal peptide fragment per se, which then is coupled to the C-terminal amino acid provided with a marker.

Useful amino protecting groups are benzyloxycarbonyl-, 9-fluorenylmethyloxycarbonyl- or t-butyloxy-carbonyl groups. To protect the guanidino group of the arginine is used protonisation, a nitroprotecting group or a p-toluenesulfonyl protecting group. The coupling between the amino acids are performed by activation of the α-carboxylic group (e.g. active esters, symmetric or asymmetric anhydrides, azide, DCCI or related reagents).

The invention will be described more in detail in the following not limited working examples, which show the preparation of different substrates according to the invention.

Purification of the intermediates and end products is performed by precipitation, crystallization or gel filtration chromatography. The purified end products are lyophilized. Prefabricated glass plates of silica gel $F_{254}$ are used in the TLC analyses. After terminated chromatography the plates are inspected in UV light (254 nm) and are then developed with ninhydrin and chlorine/dicarboxidine reagent. The $R_f$-values given are results from single chromatographies.

Solvent systems used for TLC are indicated according to the following table

| Indication | Solvent system | Volume ratio |
|---|---|---|
| A = | n-butanol: AcOH: water | (3:2:1) |
| Pa6 = | chloroform: MeOH: AcOH: water | (34:4:9:2) |
| M = | n-butanol: AcOH: water: pyridine | (15:3:12:10) |
| "3" = | EtOAc: AcOH: water: pyridine | (30:6:11:20) |

HPLC analysis were performed on Merck RP column (Hibar® LiChraCart) with 40% MeOH in 0.5% triethylaminophosphate pH = 2.35 as eluant (1 ml/min). The optical activity of the end products are determined at 589 nm in 50% acetic acid at a concentration of 0.4 - 1.0 g/100 ml and +25°C.

The below mentioned abbreviations have the following meaning: (IUPAC indication has been used where such exists).

Amino acids

Arg = arginine
D-Arg = D-arginine
Gly = glycine
Sar = sarcosine (N-methylglycine)

All amino acids in the substrates have L-configuration if nothing else is indicated.

The free amino acid or peptide is indicated by H- at the N-terminal amino group and -OH at the carboxyl terminal group. The amino group is always given to the left and the carboxyl group to the right.

Abbreviations

Ac = acetyl
AcOH = acetic acid
AMC = 7-amino-4-methylcoumarine
Boc = t-butyloxycarbonyl
Bs = benzenesulfonyl
Bz = benzoyl
Bzls = benzylsulfonyl
CHA = 3-carboxy-4-hydroxyanilide
4-ClBs = 4-chlorobenzenesulfonyl
DCCI = dicyclohexylcarbodiimide
DCU = dicyclohexylurea
DMF = dimethylformamide
Eoc = ethyloxycarbonyl
EtOAc = ethylacetate
EtOH = ethanol
$Et_3N$ = triethylamine
Ets = ethanesulfonyl
HOBT = 1-hydroxybenzotriazole
HPLC = High Performance Liquid Chromatography
I = ionic strength
Mbs = 4-methoxybenzenesulfonyl
MeOH = methanol
Mes = methanesulfonyl
Moz = 4-methoxybenzyloxycarbonyl
4-Nbs = 4-nitrobenzenesulfonyl
NEM = 4-ethylmorpholine
4-Nz = 4-nitrobenzyloxycarbonyl
Ns = β-naphtalenesulfonyl

ONp = p-nitrophenylester
pNA = p-nitroaniline
Suc = succinyl
TFA = trifluoroacetic acid
TLC = Thin Layer Chromatography
Tos = p-toluenesulfonyl
Tris = tris(hydroxymethyl)aminomethane
Z = benzyloxycarbonyl

Working examples

Example 1

Nα-Z-D-Arg-Gly-Arg-pNA·2 HCl Molecular weight = 714.62

1a) Boc-Gly-Arg-pNA·HBr Molecular weight = 532.40

43 mmol H-Arg-pNA·2HBr dissolved in 120 ml DMF is neutralized in cold (-10°C) with $Et_3N$. The $Et_3N·HBr$ formed is filtered off, whereafter 43 mmol Boc-Gly-OH, 45 mmol HOBT and 50 mmol DCCI are added. The reaction goes on during stirring for 1 hour in cold and at room temperature over the night. The DCU formed is filtered off and the solution is evaporated in vacuo to an oil which is dissolved in 160 ml EtOAc, is washed with 2% $NaHCO_3$, $H_2O$, 2% $KHSO_4$ and $H_2O$. After drying with $Na_2SO_4$ the EtOAc-phase is evaporated and precipated with diethylether.
Yield: 71%
TLC: Rf = 0.23 ($Pa_6$).

1b) H-Gly-Arg-pNA·TFA·HBr Molecular weight = 546.36

55 ml TFA is added to 30 mmol Boc-Gly-Arg-pNA·HBr (prepared according to Example 1a) dissolved in 55 ml methylenechloride. The mixture is stirred for 30 minutes at room temperature and is precipated with diethylether thereafter.
Yield: ~100%
TLC.: Rf = 0.28 (A).

1. Nα-Z-D-Arg-Gly-Arg-pNA·2HCl Molecular weight = 714.62

2.5 mmol Z-D-Arg-OH·HCl, 3 mmol HOBT and 3 mmol DCCI are added to 2.5 mmol H-Gly-Arg-pNA·TFA·HBr (prepared according to Example 1b) dissolved in 25 ml DMF and neutralized in cold (-10°C) with $Et_3N$. The mixture is stirred for 1 hour in cold and for 48 hours at room temperature. The DCU formed is filtered off and the solution is evaporated in vacuo to an oil, 30 ml water is added and the solution is washed with 2x20 ml EtOAc. The water phase is evaporated in vacuo, the product is ion exchanged on a Sephadex® QAE-25 column, in chloride form with 50% EtOH as eluent and is purified on a Merck Lobar® prepacked column (LiChroprep®. RP-8-B) with 30% MeOH as eluent (2 ml/min.) at the end. The purified product is lyophilized.
Yield: 37%
TLC: Rf = 0.11 ($Pa_6$)
HPLC: 98.5% purity
[α] = -18.1° (c=0.5%)

Example 2

Nα-Boc-D-Arg-Gly-Arg-pNA·2HCl Molecular weight = 680.59

2.5 mmol H-Gly-Arg-pNA·TFA·HBr and 2.5 mmol Boc-D-Arg-OH HCl are treated with the same coupling method and reaction conditions as in example 1.
Yield: 35%
TLC: Rf = 0.55 (M)
HPLC: 96% purity
[α] = -25.5° (c=0.4%)

Example 3

H-D-Arg-Gly-Arg-pNA·3HCl Molecular weight = 616.96

22 ml TFA is added to 12 mmol Nα-Boc-D-Arg-Gly-Arg-pNA·2HCl (prepared according to example 2) dissolved in 22 ml methylenechloride. The mixture is stirred for 30 minutes at room temperature and precipated with diethylether thereafter. The product is ion exchanged and purified in the same way as in example 1.
Yield: 38%
TLC: Rf = 0.30 (M)
HPLC: 98% purity
[α] = -62.4° (c=0.5%)

Example 4

Nα-Ets-D-Arg-Gly-Arg-pNA·2HCl Molecular weight = 672.62

0.5 mmol ethanesulfonylchloride and 80 µl $Et_3N$ are added to 0.5 mmol H-D-Arg-Gly-Arg-pNA·3HCl (prepared according to example 3) dissolved in 10 ml DMF and neutralized in cold (-10°C) with 80 µl $Et_3N$. The reaction goes on during stirring for 1 hour in cold and for 2 hours at room temperature. The mixture is evaporated in vacuo. The product is ion exchanged and purified in the same way as in example 1.
Yield: 44%
TLC: Rf = 0.5 (M)
HPLC: 97% purity
[α] = -15.6° (c=0.65%)

Example 5

Nα-Bs-D-Arg-Gly-Arg-pNA·2HCl Molecular weight = 720.66

0.5 mmol H-D-Arg-Gly-Arg-pNA·3HCl (prepared according to example 3) and 0.5 mmol benzenesulfonylchloride are treated in the same way and under the same reaction conditions as in example 4.
Yield: 37%
TLC: Rf = 0.53 (M)
HPLC: 98% purity
[α] = +11.6° (c=0.45%)

Example 6

Nα-4-Nz-D-Arg-Gly-Arg-pNA·2HCl Molecular weight = 759.64

0.5 mmol H-D-Arg-Gly-Arg-pNA·3HCl (prepared according to example 3) and 0.5 mmol 4-nitrobenzyloxycarbonylchloride are treated in the same way and under the same reaction conditions as in example 4.
Yield: 57%
TLC: Rf = 0.3 (A)
HPLC: 99% purity
[α] = -20.2°C (c=0.45%)

Example 7

Nα-4-Nbs-D-Arg-Gly-Arg-pNA·2HCl Molecular weight = 765.66

0.5 mmol H-D-Arg-Gly-Arg-pNA·3HCl (prepared according to example 3) and 0.5 mmol 4-nitrobenzenesulfonylchloride are treated in the same way and under the same reaction conditions as in example 4.
Yield: 25%
TLC: Rf = 0.28 (A)
HPLC: 97% purity
[α] = -0.8° (c=0.6%)

Example 8

$N^{\alpha}$-Tos-D-Arg-Gly-Arg-pNA·2HCl Molecular weight = 734.69

0.5 mmol H-D-Arg-Gly-Arg-pNA·3HCl (prepared according to example 3) and 0.5 mmol p-toluenesulfonylchloride are treated in the same way and under the same reaction conditions as in example 4.
Yield: 45%
TLC: Rf = 0.25 (A)
HPLC: 98% purity
[α] = +23.1° (c=0.45%)

Example 9

$N^{\alpha}$-Moz-D-Arg-Gly-Arg-pNA·2HCl Molecular weight = 744.66

0.5 mmol H-D-Arg-Gly-Arg-pNA·3HCl (prepared according to example 3) and 0.5 mmol 4-methyloxybenzyloxycarbonylazide are treated in the same way and under the same reaction conditions as in example 4.
Yield: 31%
TLC: Rf = 0.22 (A)
HPLC: 97% purity
[α] = -15.9° (c=0.4%)

Example 10

$N^{\alpha}$-Mbs-D-Arg-Gly-Arg-pNA·2HCl Molecular weight = 750.69

0.5 mmol H-D-Arg-Gly-Arg-pNA·3HCl (prepared according to example 3) and 0.5 mmol 4-methoxybenzenesulfonylchloride are treated in the same way and under the same reaction conditions as in example 4.
Yield: 45%
TLC: Rf = 0.31 (A)
HPLC: 99% purity
[α] = +22.8° (c=0.4%)

Example 11

$N^{\alpha}$-4-ClBs-D-Arg-Gly-Arg-pNA·2HCl Molecular weight = 755.12

0.5 mmol H-D-Arg-Gly-Arg-pNA·3HCl (prepared according to example 3) and 0.5 mmol 4-chlorobenzenesulfonylchloride are treated in the same way and under the same reaction conditions as in example 4.
Yield: 31%
TLC: Rf = 0.38 (A)
HPLC: 99% purity
[α] = +10.9° (c=0.4%)

Example 12

$N^{\alpha}$-Ns-D-Arg-Gly-Arg-pNA·2HCl Molecular weight = 770.73

0.5 mmol H-D-Arg-Gly-Arg-pNA·3HCl (prepared according to example 3) and 0.5 mmol β-naphtalenesulfonylchloride are treated in the same way and under the same reaction conditions as in example 4.
Yield: 25%
TLC: Rf = 0.29 (A)
HPLC: 99% purity
[α] = -21.5° (c=0.4%)

Example 13

$N^{\alpha}$-Z-D-Arg-Gly-Arg-AMC·2HCl Molecular weight = 751.70

2 mmol Z-D-Arg-Gly-OH·HCl, 2.5 mmol HOBT and 2.5 mmol DCCI are added to 2 mmol H-Arg-AMC·2HCl dissolved in 30 ml DMF and neutralized in cold (-10°C) with $Et_3N$. The reaction is performed during stirring for 1 hour in cold and for 24 hours at room temperature. The DCU formed is filtered off and the solution is evaporated in vacuo to an oil, which is ion exchanged and purified in the same way as in example 1.
Yield: 39%
TLC: Rf = 0.7 ("3")
HPLC: 99% purity
[α] = -28.6° (c=0.5%)

Example 14

$N^{\alpha}$-Ac-D-Arg-Gly-Arg-pNA·2HCl Molecular weight = 622.54

0.5 mmol H-D-Arg-Gly-Arg-pNA·3HCl (prepared according to example 3) and 0.5 mmol acetic acid anhydride are treated in the same way and under the same reaction conditions as in example 4.
Yield: 43%
TLC: Rf = 0.45 (M)
HPLC: 97% purity
[α] = -27.9° (c=0.4%)

Example 15

$N^{\alpha}$-Suc-D-Arg-Gly-Arg-pNA·2HCl Molecular weight = 680.57

0.5 mmol H-D-Arg-Gly-Arg-pNA·3HCl (prepared according to example 3) and 0.5 mmol succinic acid anhydride are treated in the same way and under the same reaction conditions as in example 4.
Yield: 40%
TLC: Rf = 0.23 (A)
HPLC: 98% purity
[α] = -21.4° (c=0.5%)

Example 16

$N^{\alpha}$-Bzls-D-Arg-Gly-Arg-pNA·2HCl Molecular weight 734.69

0.5 mmol H-D-Arg-Gly-Arg-pNA·3HCl (prepared according to example 3) and 0.5 mmol benzylsulfonylchloride are treated in the same way and under the same reaction conditions as in example 4.
Yield: 38%
TLC: Rf = 0.26 (A)
HPLC: 98% purity
[α] = -7.8 (c=0.4%)

Example 17

$N^{\alpha}$-Bz-D-Arg-Gly-Arg-pNA·2HCl Molecular weight = 684.61

17a) Z-Gly-Arg($NO_2$)-pNA Molecular weight = 530.51

0.35 mol H-Arg-($NO_2$2)pNA·HBr dissolved in 750 ml DMF is neutralized in cold (-10°C) with $Et_3N$. The $Et_3N$·HBr formed is filtered off, whereafter 0.35 mol Z-Gly-OH, 0.35 mol HOBT and 0.38 mol DCCI are added. The reaction is performed during stirring for 1 hour in cold and at room temperature over the night. The DCU formed is filtered off and the solution is evaporated in vacuo to an oil, which is triturated with 3x400 ml 2% $NaHCO_3$ and 500 ml water. The product is recrystallized from 3.5 l MeOH.
Yield: 80%

TLC: Rf = 0.6 ($Pa_6$)

17b) H-Gly-Arg($NO_2$)-pNA·HCl Molecular weight = 432.72

0.3 mol Z-Gly-Arg($NO_2$)-pNA (prepared according to example 17a) is suspended in 630 ml AcOH. 415 ml 5.6 M HBr in AcOH is added during stirring. The mixture is stirred for 45 minutes at room temperature and poured into 7.5 l dry diethylether during stirring. The precipitate is filtered, washed with diethylether and dried in vacuo.
Yield: 90%
3 g product is ion exchanged in the same way as in example 1.
Yield: 73%
TLC: Rf = 0.15 ($Pa_6$).

17c) N$^{\alpha}$-Boc-D-Arg($NO_2$)-Gly-Arg($NO_2$)-pNA Molecular weight = 697.69

4 mmol H-Gly-Arg($NO_2$)-pNA·HCl (prepared according to example 17b) dissolved in 25 ml DMF is neutralized in cold (-10°C) with $Et_3N$. The $Et_3N$·HCl formed is filtered off, whereafter 4 mmol α-Boc-D-Arg($NO_2$)-OH, 4 mmol HOBT and 4.2 mmol DCCI are added. The reaction is performed during stirring for 1 hour in cold and at room temperature over the night. The DCU formed is filtered off and the solution is evaporated in vacuo to an oil, which is suspended with 2% $NaHCO_3$, water, 2% $KHSO_4$, water and diethylether.
Yield: 73%
TLC: Rf = 0.32 ($Pa_6$)

17d) H-D-Arg($NO_2$)-Gly-Arg($NO_2$)-pNA·HCl Molecular weight = 634.04

3 mmol Nα-Boc-D-Arg-($NO_2$)-Gly-Arg($NO_2$)-pNA (prepared according to example 17c) is suspended in 11 ml AcOH, 11 ml TFA is added and the mixture is stirred for 4 hours at room temperature. The product is precipitated with diehtylether and ion exchanged in the same way as in example 1.
Yield: 72%
TLC: Rf = 0.61 (M)

17e) N$^{\alpha}$-Bz-D-Arg($NO_2$)-Gly-Arg($NO_2$)-pNA Molecular weight = 701.68

0.6 mmol benzoic anhydride and 70 µl $Et_3N$ are added to 0.5 mmol H-D-Arg($NO_2$)-Gly-Arg($NO_2$)-pNA·HCl (prepared according to example 17d) dissolved in 20 ml DMF and neutralized in cold (-10°C) with 70 µl $Et_3N$. The reaction is performed during stirring for 1 hour in cold and for 2 hours at room temperature. The mixture is evaporated in vacuo and precipitated with water. The product is suspended with 30 ml warm MeOH, is filtrated and dried.
Yield: 68%
TLC: Rf = 0.31 ($Pa_6$)

17. N$^{\alpha}$-Bz-D-Arg-Gly-Arg-pNA 2HCl Molecular weight = 684.61

0.35 mmol N$^{\alpha}$-Bz-D-Arg($NO_2$)-Gly-Arg($NO_2$)-pNA (prepared according example 17e) is deprotected by reaction with 15 ml dry HF in presence of 0.5 ml anisole in a suitable apparatus according to Sakakibara (S. Sakakibara et al. Bull. Chem. Soc., Japan vol. 240 p. 7164-67, 1967) for 60 minutes at 0°C. After terminated reaction all HF is distilled and the raw product is ion exchanged and purified in the same way as in example 1.
Yield: 36%
TLC: Rf = 0.55 (M)
HPLC: 98% purity
[α] = -24.4° (c=0,6%)

Example 18

N$^{\alpha}$-Eoc-D-Arg-Gly-Arg-pNA·2HCl Molecular weight = 652.57

18a) N$^{\alpha}$-Eoc-D-Arg($NO_2$)-Gly-Arg($NO_2$)-pNA Molecular weight = 669.63

0.6 mmol ethylchloroformate and 70 µl $Et_3N$ are added to 0.5 mmol H-D-Arg($NO_2$)-Gly-Arg($NO_2$)-pNA·HCl

(prepared according to example 17d) dissolved in 20 ml DMF and neutralized in cold (-10°C) with 70 µl $Et_3N$. The reaction goes on during stirring for 1 hour in cold and for 2 hours at room temperature. The mixture is evaporated in vacuo and precipitated with water, filtrated and washed with water and diethylether.
Yield: 89%
TLC: Rf = 0.26 ($Pa_6$)

18. N$^{\alpha}$-Eoc-D-Arg-Gly-Arg-pNA·2HCl Molecular weight = 652.57

0.45 mmol N$^{\alpha}$-Eoc-D-Arg($NO_2$)-Gly-Arg($NO_2$)-pNA (prepared according to example 18a) is deprotected by reaction with 20 ml dry HF in presence of 0.5 ml anisole in the same way as in example 17.
Yield: 41%
TLC: Rf = 0.55 (M)
HPLC: 98% purity
[α] = -25.2° (c=0.35%)

Example 19

N$^{\alpha}$-Mes-D-Arg-Gly-Arg-pNA·2HCl Molecular weight = 658.60

19a) N$^{\alpha}$-Mes-D-Arg($NO_2$)-Gly-Arg($NO_2$)-pNA Molecular weight = 675.66

0.5 mmol H-D-Arg($NO_2$)-Gly-Arg($NO_2$)-pNA·HCl (prepared according to example 17d) dissolved in 20 ml DMF and 0.6 mmol methanesulfonylchloride are treated in the same way and under the same reaction conditions as in example 17e.
Yield: 70%
TLC: Rf = 0.47 (A)

19. N$^{\alpha}$-Mes-D-Arg-Gly-Arg-pNA·2HCl Molecular weight = 658.60

0.35 mmol N$^{\alpha}$-Mes-D-Arg($NO_2$)-Gly-Arg($NO_2$)-pNA (prepared according to example 19a) is deprotected and purified in the same way as in example 17.
Yield: 43.5%
TLC: Rf = 0.48 (M)
HPLC: 97% purity
[α] = -17.6° (c=0.5%)

Example 20

N$^{\alpha}$-Z-D-Arg-Sar-Arg-pNA·2HCl Molecular weight = 728.66

20a) H-Sar-Arg-pNA·2HCl Molecular weight = 438.33

5 mmol Boc-SarOH, 5 mmol HOBT and 6 mmol DCCI are added to 5 mmol H-Arg-pNA·2HCl dissolved in 25 ml DMF and neutralized in cold (-10°C) with $Et_3N$. The mixture is stirred for 1 hour in cold and at room temperature over the night. The DCU formed is filtered off and the solution is evaporated in vacuo to an oil, which is dissolved in 100 ml n-butanol, washed with 2% $NaHCO_3$, $H_2O$, 2% $KHSO_4$ and $H_2O$. After drying with $Na_2SO_4$, the n-butanol phase is evaporated and precipitated with diethylether. The precipitate is filtrated, washed with diethylether and dried. The substance is suspended in 25 ml HCl-solution (1.5 M in AcOH) and stirred for 2 hours at room temperature, precipitated with diethylether and ion exchanged in the same way as in example 1.
Yield: 63.5%
TLC: Rf = 0.15 (A)

20. N$^{\alpha}$-Z-D-Arg-Sar-Arg-pNA·2HCl Molecular weight = 728.66

3 mmol Z-D-ArgOH·HCl, 3 mmol HOBT and 3.5 mmol DCCI are added to 3 mmol Sar-Arg-pNA·2HCl (prepared according to example 20a) dissolved in 30 ml DMF and neutralized in cold (-10°C) with $Et_3N$. The mixture is stirred for 1 hour in cold and for 72 hours at room temperature. The DCU formed is filtered off and the solution

is evaporated in vacuo. The product is ion exchanged and purified in the same way as in example 1.
Yield: 43%
TLC: Rf = 0.2 (A)
HPLC: 99% purity
[α] = -24.7° (c=0.55%)

Example 21

Nα-Z-D-Arg-Gly-Arg-CHA·2HCl Molecular weight = 729.65

21a) Boc-Arg-CHA·HCl

3.2 mmol isobutyl chloroformate is added to 3.0 mmol Boe-ArgOH dissolved in 10 ml DMF and neutralized in cold (-10°C). The reaction mixture is stirred for 15 minutes in cold and a mixture of 3 mmol 3-carboxy-4-hydroxyaniline, 3 mmol NEM and 10 ml DMF are added. The reaction goes on under stirring for 2 hours in cold and at room temperature over the night. The solution is evaporated in vacuo to an oil. 50 ml n-butanol is added and the mixture is stirred for 3 hours at room temperature. The Boc-Arg-CHA·HCl formed is filtered off.
Yield: 50%
TLC: Rf = 0.5 (A)

21b) H-Arg-CHA·HCl Molecular weight = 382.25

8 ml 2N HCl in AcOH is added to 2 mmol Boc-Arg-CHA·HCl. The reaction goes on for 45 minutes at room temperature. The reaction mixture is evaporated in vacuo to an oil, which is dissolved in isopropanol and precipitated with EtOAc.
Yield: 97%
TLC: Rf = 0.15 (A)

21c) Boc-Gly-Arg-CHA·HCl Molecular weight = 502.95

2 mmol H-Arg-CHA·2HCl dissolved in 12 ml DMF is neutralized in cold (-10°C) with NEM, whereafter 2.1 mmol Boc-Gly-ONp is added. The reaction goes on under stirring for 1 hour in cold and at room temperature over the night. The solution is evaporated in vacuo to an oil which is dissolved in 10 ml MeOH and preceipitated with EtOAc.
Yield: 88%
TLC: Rf = 0.4 (A)

21d) H-Gly-Arg-CHA·2HCl Molecular weight = 439.30

10 ml 2N HCl in AcOH is added to 2 mmol Boc-Gly-Arg-CHA HCl. The reaction mixture is stirred for 45 minutes at room temperature and then precipitated in EtOAc.
Yield 98%
TLC: Rf = 0.15 (A)

21. Nα-Z-D-Arg-Gly-Arg-CHA·2HCl Molecular weight = 729.65

1 mmol H-Gly-Arg-CHA·2HCl dissolved in 10 ml DMF is neutralized in cold (-10°C) with NEM, whereafter 1.1 mmol Z-D-Arg-ONp·$HNO_3$ is added. The reaction goes on for 1 hour in cold and for 2 hours at room temperature. The solution is evaporated in vacuo to an oil, which is precipitated with EtOAc. The product is purified on a Sephadex® column with 10% AcOH as eluent and ion exchanged on a Sephadex® QAE-25 column in chloride form with 50% EtOH as eluent.
Yield: 57%
TLC: Rf = 0.21 (A)
HPLC: 99% purity
[α] = -18.6° (C = 0.3%)

## Experiments

### Determination of enzyme kinetic properties

The kinetic data for the substrates are determined by measuring the initial velocity of hydrolysis (Vi) at different substrate concentrations (Si) and at constant enzyme concentrations (Eo). The binding constant ($K_m$) and the maximum velocity ($V_{max}$) are obtained in known manner from Lineweaver-Burk-plot, then the $K_{cat}$ is calculated (Hemker H.C. loc. cit.).

### Methods

Enzymes and substrates are mixed in a buffer solution at +37°C. The absorbance change per minute is measured at 405 nm in a spectrophotometer (pNA-substrate) or in a spectrofluorometer at excitation wavelength 380 nm and emission wavelength 440 nm (AMC-substrate).

The following commercially available reference substrates are used in the tests:

### Reference substrates

S-2222: Bz-Ile-Glu-(γ-OR)-Gly-Arg-pNA-HCl
R: H=50%; CH3=50%.
KabiVitrum AB, Stockholm, Sweden.
CBS 31.39: $CH_3SO_2$-D-Leu-Gly-Arg-pNA-AcOH
Diagnostica Stago, Asnieres s/Seine, France.

### Test 1. Determination of the kinetic constants for $FX_a$ bovine

$FX_a$ bovine (KabiVitrum) with a constant enzyme concentration
$E_0$ = 4 nmol/l (pNA - substrate)
$E_0$ = 2 nmol/l (AMC - substrate)
and the substrate in concentrations between 0.01 - 0.4 mmol/l (Si) are mixed in a trisbuffer solution 0.05 mol/l, pH = 8.3, I = 0.25 (NaCl) at 37°C. Absorbance change per minute is measured at 405 nm in a spectrophotometer for pNA - substrate and in a spectrofluorometer at emission wavelength 440 nm for AMC-substrate, respectively. From these values then $K_m$ and $K_{cat}$ are calculated, which are shown in table I.

Table I shows that the tripeptide substrates according to the invention possess a high affinity to the enzyme $FX_a$ bovine simultaneously as most of the substrates have a high rate of velocity. The selectivity constant $K_{cat}/K_m$ states that the new substrates show a better selectivity for $FX_a$ bovine than S-2222 and that even most of the new substrates are superior to CBS 31.39.

### Test 2. Determination of kinetic constants for $FX_a$ human

The substrates in concentrations between 0.1 - 0.7 mmol/l (Si) are mixed with $FX_a$ human plasma and treated according to the method as described by Aurell; L., et al. (Perspectives in Hemostatis, Ed. Fareed J. et al, New York, Pergamon Press 1981, p. 382-388).

10 µl test plasma, control or standard and 200 µl trisbuffer solution (0.05 mol/l tris, pH 7.0, I = 0.25 (NaCl)), polybrene (0.1 g/l) are mixed and incubated at 37°C for 2-4 minutes. 200 µl substrate (37°C) in concentrations between 0.1 - 0.7 mmol/l dissolved in trisbuffer solution prepared according to the above are added. The mixture is well stirred and within 30 seconds 200 µl RVV+$CaCl_2$ (20-25°C) are added and the mixture is stirred. The absorbance change is measured at once in a spectrophotometer at 405 nm and 37°C.

The results are shown in table II.

Table II shows that the new tripeptide substrates possess a superior affinity for enzyme $FX_a$ human compared with the reference substrates simultaneously as they also show a high rate of velocity, which makes them superior to the commercial available substrates.

### Test 3. Determination of thrombin sensitivity

At the determination of FX in plasma there is always a risk for formation of active thrombin which disturbes the $FX_a$ determination.

Thrombin bovine (KabiVitrum) with a constant concentration $E_0$ = 1.3 nmol/l and the substrates in concentrations between 0.1 - 0.4 mmol/l (Si) are mixed in a trisbuffer solution (0.05 mol/l, pH = 8.3, I = 0.25 (NaCl)) at 37°C. The test is performed according to test 1.

The absorbance change per minute is measured at 405 nm in a spectrophotometer and the $K_m$ and $K_{cat}$-values are calculated as showed in table III.

Table III shows that the ratio for the selectivity constant $FX_a$ bovine through thrombin for the new tripeptide substrates can be compared with the tetrapeptide and are superior to the known tripeptide when the sensitivity for $FX_a$ bovine is compared to thrombin.

Test 4. Solubility

The solubility in water and trisbuffer solution (0.05 mol/l, pH = 8.3, I = 0.25 (NaCl)), respectively at 25°C for some new substrates are shown in table IV compared with the tetrapeptide substrate S-2222.

The table shows that the new tripeptide substrates have a clearly higher solubility in water as well as in buffer solution compared with the tetrapeptide S-2222.

Table I

| Substrate Ex. no. | $K_m$ mmol/l | $K_{cat}$ $sek^{-1}$ | $K_{cat}/K_m$ l/µmol·sek |
|---|---|---|---|
| S-2222 | 0.51 | 180 | 0.35 |
| 1 | 0.11 | 250 | 2.27 |
| 2 | 0.22 | 180 | 0.82 |
| 4 | 0.19 | 270 | 1.42 |
| 5 | 0.26 | 280 | 1.07 |
| 6 | 0.21 | 350 | 1.66 |
| 7 | 0.45 | 245 | 0.55 |
| 8 | 0.22 | 230 | 1.04 |
| 9 | 0.15 | 310 | 2.06 |
| 10 | 0.20 | 210 | 1.05 |
| 11 | 0.27 | 260 | 0.96 |
| 12 | 0.12 | 140 | 1.14 |
| 13 | 0.19 | 170 | 0.89 |
| 16 | 0.04 | 110 | 2.75 |
| 18 | 0.16 | 225 | 1.41 |
| 19 | 0.17 | 200 | 1.17 |
| 20 | 0.16 | 220 | 1.37 |
| CBS 31.39 | 0.29 | 290 | 1.00 |

Table II

| Substrate Ex. no. | $K_m$ µmol/l | $V_{max}$ µmol/l min | $V_{max}/K_m$ $min^{-1}$ |
|---|---|---|---|
| S-2222 | 1970 | 46 | 0.023 |
| 1 | 380 | 73 | 0.192 |
| 4 | 340 | 46 | 0.135 |
| 9 | 210 | 51 | 0.242 |
| 16 | 50 | 15 | 0.300 |
| CBS 31.39 | 1060 | 61 | 0.057 |

Tabell III

| Substrate Ex. no. | $K_m$ mmol/l | $K_{cat}$ $sek^{-1}$ | $K_{cat}/K_m$ 1/µmol·sek | $K_{cat}/K_m$ $FX_a$ bovine / $K_{cat}/K_m$ thrombin |
|---|---|---|---|---|
| S-2222 | 0.71 | 13 | 0.018 | 19.4 |
| 1 | 0.13 | 13 | 0.100 | 22.7 |
| 4 | 0.41 | 23 | 0.056 | 25.4 |
| 9 | 0.073 | 11 | 0.151 | 13.6 |
| 16 | 0.16 | 17 | 0.106 | 25.9 |
| CBS 31.39 | 0.23 | 72 | 0.313 | 3.2 |

Table IV

| Substrate Ex. no. | Relative solubility | |
|---|---|---|
| | water | tris-buffer |
| S-2222 | 1 | 1 |
| 1 | >7 | > 5 |
| 4 | >7 | >10 |
| 9 | > 3 | > 3 |
| 16 | > 5 | >10 |

## Claims

**Claims for the following Contracting States: BE, CH, DE, FR, GB, IT, LI, LU, NL, SE**

1. Tripeptide derivatives having the general formula

$$R_1\text{-X-D-Arg-A-Arg-NH-}R_2$$

wherein $R_1$ is hydrogen, α- or β-naphtyl residue, lower alkyl residue which may be substituted with a carboxyl group, unsubstituted or substituted phenyl- or phenylalkyl residue, wherein the alkyl group having 1 to 4 carbon atoms and preferably wherein the substitution is in para position in the phenyl ring and substituted with a lower alkyl, lower alkoxy, halogen or nitro group.

X = 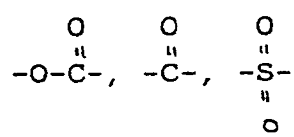

or a single bond with the proviso that when

$R_1$ is hydrogen then and only then X is a single bond.

A = Gly or Sar

$R_2$ = an aromatic or heterocyclic residue which gives a compound $R_2$-$NH_2$ by enzymatic hydrolysis, which can be determined quantitatively

or disalt and trisalts of inorganic or organic acids thereof.

2. Tripeptide derivatives according to Claim 1, characterized by that A is Gly.

3. Tripeptide derivatives according to any of claims 1-2, characterized by that X is a oxycarbonyl or sulfonyl group.

4. Tripeptide derivatives according to any of claims 1-3, characterized by that $NH_2$-$R_2$ is a chromogenic or a fluorogenic group.

5. Tripeptide derivatives according to any of claim 4, characterized by that $NH_2$-$R_2$ is p-nitroaniline, 7-amino-4-methylcoumarine or 3-carboxy-4-hydroxyanilide.

6. Tripeptide derivatives according to any of claims 1-5, characterized by that $R_1$ is ethyl,benzyl or tert.butyl.

7. Tripeptide derivatives according to any of claims 1-6, characterized by that the derivative is isolated in form of a disalt.

8. Tripeptide derivatives according to claim 1, characterized by the tripeptide derivative being $N^{\alpha}$-benzylsulfonyl-D-Arg-Gly-Arg-p-nitroaniline.2HCl.

9. Tripeptide derivatives according to claim 1, characterized by the tripeptide derivative being $N^{\alpha}$-4-methoxybenzyloxycarbonyl-D-Arg-Gly-Arg-p-nitroaniline.2HCl.

10. Tripeptide derivatives according to claim 1, characterized by the tripeptide derivative being $N^{\alpha}$-ethanesulfonyl-D-Arg-Gly-Arg-p-nitroaniline.2HCl.

11. Tripeptide derivatives according to claim 1, characterized by the tripeptide derivative being $N^{\alpha}$-benzyloxycarbonyl-D-Arg-Gly-Arg-p-nitroaniline.2HCl.

12. Tripeptide derivatives according to claim 1, characterized by the tripeptide derivative being $N^{\alpha}$-t-butyloxycarbonyl-D-Arg-Gly-Arg-p-nitroaniline.2HCl.

13. Process for the preparation of tripeptide derivatives, characterized in that the synthesis is performed step-by-step by coupling to Arg which have -NH-$R_2$, or a deprotectable carboxyl protecting group which then is exchanged by -NH-$R_2$, or by coupling of $R_1$-X-D-Arg-A to Arg-NH-$R_2$.

14. Method for determination of serine proteases, especially Factor $X_a$, characterized by the use of a peptide derivatives according to any of claims 1-12.

15. Method for determination of components which can interact with serine proteases or zymogen forms thereof, characterized by the use of peptide derivatives according to any of claims 1-12.

16. Method according to claims 14 or 15 for determination of FX, FVII, $FVII_a$, FVIII:C, FVIII:$C_a$, FIX, $FIX_a$, antithrombin III, platelet factor 4, heparin, low molecular weight heparins and heparinoids.

**Claims for the following Contracting State: AT**

1. Process for the preparation of a tripeptide derivative having the general formula

$$R_1\text{-X-D-Arg-A-Arg-NH-}R_2$$

wherein $R_1$ is hydrogen, α- or β-naphtyl residue, lower alkyl residue which may be substituted with a carboxyl group, unsubstituted or substituted phenyl- or phenylalkyl residue, wherein the alkyl group having 1 to 4 carbon atoms and preferably wherein the substitution is in para position in the phenyl ring and substituted with a lower alkyl, lower alkoxy, halogen or nitro group.

$$X = -O-\overset{\overset{O}{\|}}{C}-,\ -\overset{\overset{O}{\|}}{C}-,\ -\underset{\underset{O}{\|}}{\overset{\overset{O}{\|}}{S}}-$$

or a single bond with the proviso that when

$R_1$ is hydrogen then and only then X is a single bond.

A = Gly or Sar

$R_2$ = an aromatic or heterocyclic residue which gives a compound $R_2$-$NH_2$ by enzymatic hydrolysis, which can be determined quantitatively

or disalt and trisalts of inorganic or organic acids thereof,

characterized in that the synthesis is performed step-by-step by coupling to Arg which have -NH-$R_2$, or a deprotectable carboxyl protecting group which then is exchanged by -NH-$R_2$, or by coupling of $R_1$-X-D-Arg-A to Arg-NH-$R_2$.

2. Process according to Claim 1, characterized by that A is Gly.

3. Process according to any of claims 1-2, characterized by that X is a oxycarbonyl or sulfonyl group.

4. Process according to any of claims 1-3, characterized by that $NH_2$-$R_2$ is a chromogenic or a fluorogenic group.

5. Process according to any of claim 4, characterized by that $NH_2$-$R_2$ is p-nitroaniline, 7-amino-4-methylcoumarine or 3-carboxy-4-hydroxyanilide.

6. Process according to any of claims 1-5, characterized by that $R_1$ is ethyl, benzyl or tert.butyl.

7. Process according to any of claims 1-6, characterized by that the derivative is isolated in form of a disalt.

8. Process according to claim 1, characterized by the tripeptide derivative being $N^{\alpha}$-benzyl-sulfonyl-D-Arg-Gly-Arg-p-nitroaniline.2HCl.

9. Process according to claim 1, characterized by the tripeptide derivative being $N^{\alpha}$-4-methoxybenzyloxycarbonyl-D-Arg-Gly-Arg-p-nitroaniline.2HCl.

10. Process according to claim 1, characterized by the tripeptide derivative being $N^{\alpha}$-ethanesulfonyl-D-Arg-Gly-Arg-p-nitroaniline.2HCl.

11. Process according to claim 1, characterized by the tripeptide derivative being $N^{\alpha}$-benzyloxycarbonyl-D-Arg-Gly-Arg-p-nitroaniline.2HCl.

12. Process according to claim 1, characterized by the tripeptide derivative being $N^{\alpha}$-t-butyloxycarbonyl-D-Arg-Gly-Arg-p-nitroaniline.2HCl.

13. Method for determination of serine proteases, especially Factor $X_a$, characterized by the use of a peptide derivatives produced according to any of claims 1-12.

14. Method for determination of components which can interact with serine proteases or zymogen forms thereof, characterized by the use of peptide derivatives produced according to any of claims 1-12.

15. Method according to claims 13 or 14 for determination of FX, FVII, $FVII_a$, FVIII:C, FVIII:$C_a$, FIX, $FIX_a$, antithrombin III, platelet factor 4, heparin, low molecular weight heparins and heparinoids.

## Patentansprüche

### Patentansprüche für folgende Vertragsstaaten: BE, CH, DE, FR, GB, IT, LI, LU, NL, SE

1. Tripeptid-Derivate, gekennzeichnet durch die allgemeine Formel

$$R_1 - X - D\text{-}Arg - A - Arg - NH - R_2$$

in der

$R_1$ Wasserstoff, einen alpha- oder beta-Naphthylrest, einen niedrigen Alkylrest, der mit einer Carboxylgruppe substituiert sein kann, oder einen unsubstituierten oder substituierten Phenyl- oder Phenylalkylrest, in dem die Alkylgruppe 1 - 4 Kohlenstoffatome aufweist, wobei vorzugsweise die Substitution in dem Phenylring in para-Stellung und mit einer niedrigen Alkyl-, niedrigen Alkoxy-, Halogen- oder Nitrogruppe erfolgt ist,

X = 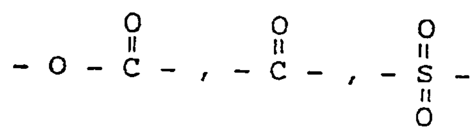

oder eine Einfachbindung, unter der Voraussetzung, daß dann und nur dann X eine Einfachbindung bedeutet, wenn $R_1$ Wasserstoff ist,

A = Gly oder Sar, und

$R_2$= einem aromatischen oder heterocyclischen, unter enzymatischer Hydrolyse eine quantitativ bestimmbare Verbindung $R_2$-$NH_2$ ergebenden Rest bedeuten, oder Disalze oder Trisalze derselben mit anorganischen oder organischen Säuren.

2. Tripeptid-Derivate nach Anspruch 1, dadurch gekennzeichnet, daß A Gly ist.

3. Tripeptid-Derivate nach Anspruch 1 oder 2, dadurch gekennzeichnet, daß X eine Oxycarbonyl- oder Sulfonylgruppe ist.

4. Tripeptid-Derivate nach mindestens einem der Ansprüche 1 bis 3, dadurch gekennzeichnet, daß $NH_2$-$R_2$ eine chromogene oder fluorogene Gruppe ist.

5. Tripeptid-Derivate nach Anspruch 4, dadurch gekennzeichnet, daß $NH_2$-$R_2$ p-Nitroanilin, 7-Amino-4-methylcumarin oder 3-Carboxy-4-hydroxyanilid ist.

6. Tripeptid-Derivate nach mindestens einem der Ansprüche 1 bis 5, dadurch gekennzeichnet, daß $R_1$ Ethyl, Benzyl oder tert.-Butyl ist.

7. Tripeptid-Derivate nach mindestens einem der Ansprüche 1 bis 6, dadurch gekennzeichnet, daß das Derivat in Form eines Disalzes isoliert worden ist.

8. Tripeptid-Derivate nach Anspruch 1, dadurch gekennzeichnet, daß das Tripeptid-Derivat $N^{\alpha}$ - Benzylsulfonyl - D - Arg - Gly - Arg - p-Nitroanilin · 2HCl ist.

9. Tripeptid-Derivate nach Anspruch 1, dadurch gekennzeichnet, daß das Tripeptid-Derivat $N^{\alpha}$ - 4-Methoxybenzyloxycarbonyl - D - Arg - Gly - Arg - p-Nitroanilin · 2HCl ist.

10. Tripeptid-Derivate nach Anspruch 1, dadurch gekennzeichnet, daß das Tripeptid-Derivat $N^{\alpha}$ - Ethansulfonyl - D - Arg - Gly - Arg - p-Nitroanilin · 2HCl ist.

11. Tripeptid-Derivate nach Anspruch 1, dadurch gekennzeichnet, daß das Tripeptid-Derivat $N^{\alpha}$ - Benzyloxycarbonyl - D - Arg - Gly - Arg - p-Nitroanilin · 2HCl ist.

12. Tripeptid-Derivate nach Anspruch 1, dadurch gekennzeichnet, daß das Tripeptid-Derivat $N^{\alpha}$ - t-Butyloxycarbonyl - D - Arg - Gly - Arg - p-Nitroanilin · 2HCl ist.

13. Verfahren zur Herstellung von Tripeptid-Derivaten, dadurch gekennzeichnet, daß man die Synthese

stufenweise durch Ankopplung an Arg, das eine Gruppe -NH-$R_2$ oder eine abspaltbare Carboxylschutzgruppe, welche durch -NH-$R_2$ austauschbar ist, aufweist, oder durch Kopplung von $R_1$ - X - D - Arg - A an Arg - NH - $R_2$ durchführt.

14. Verfahren zur Bestimmung von Serinproteasen, insbesondere Faktor $X_a$, dadurch gekennzeichnet, daß man Peptid-Derivate gemäß einem der Ansprüche 1 bis 12 verwendet.

15. Verfahren zur Bestimmung von Komponenten, die mit Serinproteasen oder Zymogenformen derselben wechselwirken können, dadurch gekennzeichnet, daß man Peptid-Derivate gemäß einem der Ansprüche 1 bis 12 verwendet.

16. Verfahren gemäß Anspruch 14 oder 15 zur Bestimmung von FX, FVII, $FVII_a$, FVIII:C, FIIIV:$C_a$, FIX, $FIX_a$, Antithrombin III, Plättchenfaktor 4, Heparin, Heparinen mit niedrigem Molekulargewicht und Heparinoiden.

**Patentansprüche für folgenden Vertragsstaat: AT**

1. Verfahren zur Herstellung von Tripeptid-Derivaten, gekennzeichnet durch die allgemeine Formel

$$R_1 - X - D\text{-}Arg - A - Arg - NH - R_2$$

in der

$R_1$ Wasserstoff, einen alpha- oder beta-Naphthylrest, einen niedrigen Alkylrest, der mit einer Carboxylgruppe substituiert sein kann, oder einen unsubstituierten oder substituierten Phenyl- oder Phenylalkylrest, in dem die Alkylgruppe 1 - 4 Kohlenstoffatome aufweist, wobei vorzugsweise die Substitution in dem Phenylring in para-Stellung und mit einer niedrigen Alkyl-, niedrigen Alkoxy-, Halogenoder Nitrogruppe erfolgt ist,

$$X = -O-\overset{\overset{\displaystyle O}{\|}}{C}- \;,\; -\overset{\overset{\displaystyle O}{\|}}{C}- \;,\; -\underset{\underset{\displaystyle O}{\|}}{\overset{\overset{\displaystyle O}{\|}}{S}}-$$

oder eine Einfachbindung, unter der Voraussetzung, daß dann und nur dann X eine Einfachbindung bedeutet, wenn $R_1$ Wasserstoff ist,

A = Gly oder Sar, und

$R_2$= einem aromatischen oder heterocyclischen, unter enzymatischer Hydrolyse eine quantitativ bestimmbare Verbindung $R_2$-$NH_2$ ergebenden Rest bedeuten, oder von Disalzen oder Trisalzen derselben mit anorganischen oder organischen Säuren, dadurch gekennzeichnet, daß man die Synthese stufenweise durch Ankopplungan Arg, das eine Gruppe -NH-$R_2$ oder eine abspaltbare Carboxylschutzgruppe, welche durch -NH-$R_2$ austauschbar ist, aufweist, oder durch Kopplung von $R_1$ - X - D - Arg - A an Arg - NH - $R_2$ durchführt.

2. Verfahren nach Anspruch 1, dadurch gekennzeichnet, daß A Gly ist.

3. Verfahren nach Anspruch 1 oder 2, dadurch gekennzeichnet, daß X eine Oxycarbonyl- oder Sulfonylgruppe ist.

4. Verfahren nach mindestens einem der Ansprüche 1 bis 3, dadurch gekennzeichnet, daß $NH_2$-$R_2$ eine chromogene oder fluorogene Gruppe ist.

5. Verfahren nach Anspruch 4, dadurch gekennzeichnet, daß $NH_2$-$R_2$ p-Nitroanilin, 7-Amino-4-methylcumarin oder 3-Carboxy-4-hydroxyanilin ist.

6. Verfahren nach mindestens einem der Ansprüche 1 bis 3, dadurch gekennzeichnet, daß $R_1$ Ethyl, Benzyl oder tert.-Butyl ist.

7. Verfahren nach mindestens einem der Ansprüche 1 bis 6, dadurch gekennzeichnet, daß das Tripeptid-Derivat in Form seines Disalzes isoliert wird.

8. Verfahren nach Anspruch 1, dadurch gekennzeichnet, daß das Tripeptid-Derivat $N^\alpha$-Benzyl-sulfonyl-D-Arg-Gly-Arg-p-Nitroanilin·2HCl ist.

9. Verfahren nach Anspruch 1, dadurch gekennzeichnet, daß das Tripeptid-Derivat $N^\alpha$-4-Methoxybenzyloxycarbonyl-D-Arg-Gly-Arg-p-Nitroanilin·2HCl ist.

10. Verfahren nach Anspruch 1, dadurch gekennzeichnet, daß das Tripeptid-Derivat $N^\alpha$-Ethansulfonyl-D-Arg-Gly-Arg-p-Nitroanilin·2HCl ist.

11. Verfahren nach Anspruch 1, dadurch gekennzeichnet, daß das Tripeptid-Derivat $N^\alpha$-Benzyloxycarbonyl-D-Arg-Gly-Arg-p-Nitroanilin·2HCl ist.

12. Verfahren nach Anspruch 1, dadurch gekennzeichnet, daß das Tripeptid-Derivat $N^\alpha$-Butyloxycarbonyl-D-Arg-Gly-Arg-p-Nitroanilin·2HCl ist.

13. Verfahren zur Bestimmung von Serinproteasen, insbesondere Faktor $X_a$, dadurch gekennzeichnet, daß man Peptid-Derivate gemäß einem der Ansprüche 1 bis 12 verwendet.

14. Verfahren zur Bestimmung von Komponenten, die mit Serinproteasen oder Zymogenformen derselben wechselwirken können, dadurch gekennzeichnet, daß man Peptid-Derivate gemäß einem der Ansprüche 1 bis 12 verwendet.

15. Verfahren gemäß Anspruch 13 oder 14 zur Bestimmung von FX, FVII, $FVII_a$, FVIII:C, FIIIV:$C_a$, FIX, $FIX_a$, Antithrombin III, Plättchenfaktor 4, Heparin, Heparinen mit niedrigem Molekulargewicht und Heparinoiden.

## Revendications

### Revendications pour les Etats contractants suivants: BE, CH, DE, FR, GB, IT, LI, LU, NL, SE

1. Dérivés de tripeptides, caractérisés en ce qu'ils répondent à la formule générale :

$$R_1 - X - D\text{-}Arg - A - Arg - NH - R_2$$

dans laquelle $R_1$ représente l'hydrogène, un résidu α- ou β-naphtyle, un résidu alkyle inférieur qui peut être substitué avec un groupe carboxyle, un résidu phényle ou phénylalkyle non substitué ou substitué, dans lequel le groupe alkyle possède 1 à 4 atomes de carbone et de préférence dans lequel la substitution est en position para dans le noyau phényle, le substituant étant un groupe alkyle inférieur, alkoxy inférieur, halogéno ou nitro, X représente un groupe

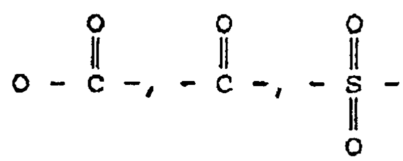

ou une liaison simple, sous réserve que, lorsque $R_1$ représente l'hydrogène, X représente alors, et seulement alors, une liaison simple,

A représente Gly ou Sar,

$R_2$ représente un résidu aromatique ou hétérocyclique qui donne un composé de formule $R_2$-$NH_2$ par hydrolyse enzymatique, qui peut être dosé quantitativement, ou des sels doubles et des sels triples d'acides inorganiques ou organiques de ces dérivés.

2. Dérivés de tripeptides suivant la revendication 1, caractérisés en ce que A représente Gly.

3. Dérivés de tripeptides suivant l'une quelconque des revendications 1 et 2, caractérisés en ce que X représente un groupe oxycarbonyle ou sulfonyle.

4. Dérivés de tripeptides suivant l'une quelconque des revendications 1 à 3, caractérisés en ce que le groupe $NH_2$-$R_2$ est un groupe chromogène ou fluorogène.

5. Dérivés de tripeptides suivant l'une quelconque des revendications 1 à 4, caractérisés en ce que $NH_2$-$R_2$ est la p-nitroaniline, la 7-amino-4-méthylcoumarine ou le 3-carboxy-4-hydroxyanilide.

6. Dérivés de tripeptides suivant l'une quelconque des revendications 1 à 5, caractérisés en ce que $R_1$ représente un groupe éthyle, benzyle ou tertio-butyle.

7. Dérivés de tripeptides suivant l'une quelconque des revendications 1 à 6, caractérisés en ce que le dérivé est isolé sous forme d'un sel double.

8. Dérivé de tripeptide suivant la revendication 1, caractérisé en ce qu'il s'agit du $N^α$-bensylsulfonyl-D-Arg-Gly-Arg-p-nitroaniline.2HCl.

9. Dérivé de tripeptide suivant la revendication 1, caractérisé en ce qu'il s'agit du $N^α$-4-méthoxybenzyloxycarbonyl-D-Arg-Gly-Arg-p-nitroaniline.2HCl.

10. Dérivé de tripeptide suivant la revendication 1, caractérisé en ce qu'il s'agit du $N^α$-éthanesulfonyl-D-Arg-Gly-Arg-p-nitroaniline.2HCl.

11. Dérivé de tripeptide suivant la revendication 1, caractérisé en ce qu'il s'agit du $N^α$-benzyloxycarbonyl-D-Arg-Gly-Arg-p-nitroaniline.2HCl.

12. Dérivé de tripeptide suivant la revendication 1, caractérisé en ce qu'il s'agit du $N^α$-tertiobutyloxycarbonyl-D-Arg-Gly-Arg-p-nitroaniline.2HCl.

13. Procédé de préparation de dérivés de tripeptides suivant l'une quelconque des revendications 1 à 12, caractérisé en ce que la sunthèse est conduite étape par étape par couplage à Arg qui possède un gourpe -NH-$R_2$, ou un groupe protecteur du groupe carboxyle pouvant être éliminé qui est ensuite échangé avec -NH-$R_2$, ou par couplage de $R_1$-X-D-Arg-A à Arg-NH-$R_2$.

14. Procédé de dosage de sérine-protéases, notamment du Facteur $X_a$, caractérisé en ce qu'il consiste à

utiliser des dérivés de peptides suivant l'une quelconque des revendications 1 à 12.

15. Procédé de dosage de constituants qui peuvent interagir avec des sérine-protéases ou des zymogènes de ces sérine-protéases, caractérisé en ce qu'il consiste à utiliser des dérivés de peptides suivant l'une quelconque des revendications 1 à 12.

16. Procédé suivant la revendication 14 ou 15 pour le dosage des facteurs FX, FVII, $FVII_a$, FVIII:C, $FVIII:C_a$, FIX, $FIX_a$, de l'antithrombine III, du facteur plaquettaire 4, de l'héparine, d'héparine de bas poids moléculaire et d'héparinoïdes.

**Revendications pour l'Etat contractant suivant: AT**

1. Procédé de préparation d'un dérivé de tripeptide répondant à la formule générale :

$$R_1 - X - D - Arg - A - Arg - NH - R_2$$

dans laquelle $R_1$ représente l'hydrogène, un résidu α- ou β-naphtyle, un résidu alkyle inférieur qui peut être substitué avec un groupe carboxyle, un résidu phényle ou phénylalkyle non substitué ou substitué, dans lequel le groupe alkyle possède 1 à 4 atomes de carbone et de préférence dans lequel la substitution est en position para dans le noyau phénule, le substituant étant un groupe alkyle inférieur, alkoxy inférieur, halogéno ou nitro, X représente un groupe

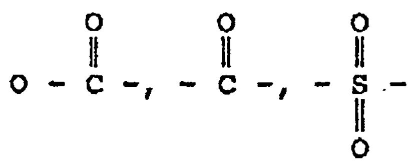

ou une liaison simple, sous réserve que, lorsque $R_1$ représente l'hydrogène, X représente alors, et seulement alors, une liaison simple,

A représente Gly ou Sar,

$R_2$ représente un résidu aromatique ou hétérocyclique qui donne un composé de formule $R_2$-$NH_2$ par hydrolyse enzymatique, qui peut être dosé quantitativement, ou de sels doubles et de sels triples d'acides inorganiques ou organiques de ce dérivé, caractérisé en ce que la synthèse est effectuée étape par étape par couplage à Arg qui possède un groupe - NH - $R_2$, ou un groupe protecteur du groupe carboxyle pouvant être éliminé, qui est ensuite échangé avec - NH - $R_2$, ou par couplage de $R_1$ - X - D - Arg - A à Arg - NH - $R_2$.

2. Procédé suivant la revendication 1, caractérisé en ce que A représente Gly.

3. Procédé suivant l'une quelconque des revendications 1 et 2, caractérisé en ce que X représente un groupe oxycarbonyle ou sulfonyle.

4. Procédé suivant l'une quelconque des revendications 1 à 3, caractérisé en ce que le groupe $NH_2$-$R_2$ est un groupe chromogène ou fluorogène.

5. Procédé suivant l'une quelconque des revendications 1 à 4, caractérisé en ce que $NH_2$-$R_2$ représente la p-nitroaniline, la 7-amino-4-méthylcoumarine ou le 3-carboxy-4-hydroxyanilide.

6. Procédé suivant l'une quelconque des revendications 1 à 5, caractérisé en ce que $R_1$ représente un groupe éthyle, benzyle ou tertio-butyle.

7. Procédé suivant l'une quelconque des revendications 1 à 6, caractérisé en ce que le dérivé de tripeptide est sous forme d'un sel double.

8. Procédé suivant la revendication 1, caractérisé en ce que le dérivé de tripeptide est le $N^{\alpha}$-benzylsulfonyl-D-Arg-Gly-Arg-p-nitroaniline.2HCl.

9. Procédé suivant la revendication 1, caractérisé en ce que le dérivé de tripeptide est le $N^{\alpha}$-4-méthoxybensyloxycarbonyl-D-Arg-Gly-Arg-p-nitroaniline. 2HCl.

10. Procédé suivant la revendication 1, caractérisé en ce que le dérivé de tripeptide est le $N^{\alpha}$-éthanesulfonyl-D-Arg-Gly-Arg-p-nitroaniline.2HCl.

11. Procédé suivant la revendication 1, caractérisé en ce que le dérivé de tripeptide est le $N^{\alpha}$-benzyloxycarbonyl-D-Arg-Gly-Arg-p-nitroaniline.2HCl.

12. Procédé suivant la revendication 1, caractérisé en ce que le dérivé de tripeptide est le $N^{\alpha}$-tertio-butyloxycarbonyl-D-Arg-Gly-Arg-p-nitroaniline.2HCl.

13. Procédé de dosage de sérine-protéases, notamment du Facteur $X_a$, caractérisé en ce qu'il consiste à utiliser un dérivé de peptide produit suivant l'une quelconque des revendications 1 à 12.

14. Procédé de dosage de constituants qui peuvent interagir avec des sérine-protéases ou des zymogènes

de ces sérine-protéases, caractérisé en ce qu'il consiste à utiliser un dérivé de peptide produit suivant l'une quelconque des revendications 1 à 12.

15. Procédé suivant la revendication 13 ou 14, pour le dosage des facteurs FX, FVII, $FVII_a$, FVIII:C, $FVIII:C_a$, FIX, $FIX_a$, de l'antithrombine III, du facteur plaquettaire 4, de l'héparine, d'héparines de bas poids moléculaire et d'héparinoïdes.